# EUROPÄISCHE PATENTSCHRIFT

(11) **EP 0 304 784 B1**
(45) Veröffentlichungstag und Bekanntmachung des Hinweises auf die Patenterteilung: **10.11.1993**
(21) Anmeldenummer: 88113325.0
(22) Anmeldetag: 17.08.1988
(51) Int. Cl.: C07C 69/54, C07C 67/14

(54) **Verfahren zur Herstellung von Bisphenol-bisacrylaten**
Process for the preparation of bisphenol bisacrylates
Procédé de préparation de bisphénol-bisacrylates

(30) Priorität: 25.08.1987 DE 3728302
(43) Veröffentlichungstag der Anmeldung: 01.03.1989
(73) Patentinhaber: BAYER AG, 51368 Leverkusen (DE)
(72) Erfinder: Buysch, Hans-Josef, Dr., D-4150 Krefeld (DE); Klöker, Werner, Prof. Dr., D-4150 Krefeld (DE)

(56) Entgegenhaltungen:
- AU-A- 434 609
- DE-A- 1 543 537
- FR-A- 1 089 031

## Beschreibung

Die Erfindung betrifft ein Verfahren zur Herstellung von Bisphenol-bisacrylaten durch Umsetzung von Bisphenolen mit Acrylsäurehalogeniden in Gegenwart von Basen.

Bisphenolbisacrylate, z.B. die Bisacrylate von Bisphenol A sind bekannt. Sie sind Ausgangsprodukte zur Herstellung von vernetzten Polymeren. Sie polymerisieren sehr leicht, was bei ihrer Herstellung und Reinigung zu unerwünschten Nebenreaktionen führt. Da auch durch Zusatz von Inhibitoren eine unkontrollierte Polymerisation ablaufen kann, ist ihre Herstellung, insbesondere im technischen Maßstab, schwierig (z.B. GB-PS 738 954).

Aus der GB-PS 738 954 ist weiterhin bekannt, daß bei niedrigen Temperaturen (unter 50°C) die Nebenreaktionen zurückgedrängt werden können. So wird z.B. bei der Herstellung von Bisphenol A-Bismethacrylat aus Methacryloylchlorid mit dem wasserfreien Dinatriumsalz von Bisphenol A in Benzol eine Ausbeute von 53 % d.Th. erzielt.

Aus der JP-OS 73-48453 ist bekannt, Bisphenol A-Bismethacrylat aus Methacrylsäure und Bisphenol A in siedendem Benzol unter Katalyse mit HCl-Gas in hohen Ausbeuten herzustellen. Jedoch ist vor allem die Übertragung dieses Verfahrens auf technische Ansatzmengen nicht unproblematisch, da Bisphenol in Gegenwart von starken Säuren bei erhöhten Temperaturen leicht gespalten und isomerisiert wird.

Weiterhin ist bekannt die Kondensation von Säurehalogenid und phenolischer Verbindung in disperser Phase aus Wasser und einem mit Wasser nicht mischbaren organischen Lösungsmittel in Gegenwart von anorganischen Basen, wie Natriumhydroxid durchzuführen (z.B. J. Amer. Chem. Soc. 81, 4310-3 (1959)). Mit diesem Verfahren können jedoch Bisphenole nicht quantitativ zu Bisacrylaten umgesetzt werden, da infolge partieller Hydrolyse des Acryloylchlorids durch die basische wäßrige Lösung immer auch der Halbester des Bisphenols neben dem Bisester gebildet wird. Da dieser nicht leicht zu entfernen ist, stört er als phenolische inhibierende Substanz die Polymerisation des Bisacrylates. Die Isolierung des Reaktionsproduktes aus der Lösung durch Eindampfen ist immer, auch bei schonendem Vorgehen von partieller Polymerisation begleitet.

Aus der US-PS 4 068 082 ist bekannt, Bisphenolacrylate in polaren aprotischen, Solventien, wie Aceton, in Gegenwart von Aminen herzustellen. Die Abtrennung des Amins und Aminsalzes aus der Reaktionsmischung ist aufwendig und es fallen große Mengen an Lösungsmitteln an, die aufgearbeitet werden müssen, da das Reaktionsgemisch auf eine im Verhältnis zur Reaktionsmischung riesige Menge Eiswasser gegossen wird.

Aus DE-OS 15 43 537 ist ein Verfahren zur Herstellung von Estern einwertiger, halogenierter Phenole durch Umsetzung mit Säurechloriden bekannt, bei dem in Gegenwart von nur katalytischen Mengen tertiärer Amine gearbeitet wird und entstandener Chlorwasserstoff durch N₂ aus dem Reaktionsgemisch ausgeblasen wird. Es sind lange Reaktionszeiten und hohe Temperaturen erforderlich, bei denen polymerisationsfähige Stoffe hohe Verluste erleiden.

Es wurde nun gefunden, daß die Herstellung von Bisphenolbisacrylaten aus Acryloylchloriden und Bisphenolen in Gegenwart von tertiären Aminen in unpolaren Lösungsmitteln mit guten Ausbeuten an Bisphenol-Bisacrylat durchgeführt werden kann unter gleichzeitiger Vermeidung der oben geschilderten Probleme.

Gegenstand der Erfindung ist ein Verfahren zur Herstellung von Bisphenol-bisacrylaten durch Umsetzung von Bisphenolen mit Acrylsäurehalogeniden in Gegenwart von Basen, dadurch gekennzeichnet, daß man die Umsetzung in einem unpolaren Lösungsmittel in Anwesenheit eines tertiären Amins und in Abwesenheit einer wäßrigen Phase durchführt, wobei das Molverhältnis Bisphenol, Acrylsäurehalogenid und Amin 1:2:2 bis 1:2,2:2,4 beträgt, anschließend die Aminsalze durch Extraktion mit Wasser entfernt und die Bisphenolbisacrylate aus der organischen Phase kristallin gewinnt.

Die Salze aus Bisphenolen und tertiären Aminen sind in unpolaren Solventien löslich, zumindest geringfügig löslich, obwohl die Bisphenole selbst darin praktisch unlöslich sind.

Geeignete Bisphenole sind solche mit 2 und 3 aromatischen Kernen wie 4,4'-Dihydroxy-diphenyl, 4,4'-Dihydroxy-diphenylether, 4,4'-Dihydroxydiphenylsulfid, Dihydroxydiphenylsulfon, Dihydroxydiphenylethan, Dihydroxydiphenylmethan und halogensubstituierte Bisphenole wie Tetrachlor- und Tetrabrombisphenol A, bevorzugt 4,4′-Dihydroxy-3,3′-diphenyl-diphenylpropan, 4,4′-Dihydroxy-3,5,3′,5′-tetramethyldiphenylpropan, 4,4′-Dihydroxy-3,5,3′,5′-tetramethyldiphenylmethan, 5,5′-Dihydroxy-1,1,1′,1′-tetramethyl-spiro-bisindan, 5-Hydroxy-1,1,3-(4′-hydroxyphenyl)-indan, 4,4′-Dihydroxydiphenylethan, 4,4′-Dihydroxydiphenyl-cyclohexan-1,1, α,α′-Bis-(4-hydroxyphenyl)-m- und -p-diisopropylbenzol, besonders bevorzugt Bisphenol A.

Bevorzugte Acrylsäurederivate sind Acryloylhalogenide wie Acryloylbromid, Acryloylchlorid, Methacryloylbromid, besonders bevorzugt Methacryloylchlorid.

Erfindungsgemäß einsetzbare Amine sind tertiäre Amine wie heterocyclische und aliphatische Amine mit bis zu 15 C-Atomen, beispielsweise Pyridin, α-, β-, γ-Picolin, N-Methylmorpholin, Diethylcyclohexylamin, Isopropyldiethylamin, Dimethylbutylamin, bevorzugt sind aliphatische Amine mit bis zu 12 C-Atomen wie Diisopropylamin, Tripropylamin, Tributylamin, bevorzugt Triethylamin.

Unpolare Lösungsmittel im Sinne der Erfindung sind mit Wasser nicht mischbare Verbindungen mit 4 bis 10, bevorzugt 5 bis 8 Kohlenstoffatomen, von denen wenigstens 2 aliphatisch sind, beispielsweise Tetralin, Isopropylbenzol, Decalin, Di-tert.-butylether, Diisobutylen, Triisopropylen und Decan, bevorzugt Cycloheptan, Cyclooctan, Isopropylcyclohexan und Ethylcyclohexan, besonders bevorzugt Pentane, Hexane, Petrolether, Ligroin, Isooctan, n-Octan, Cyclohexan, Methylcyclohexan und Methylcyclopentan.

Als Reaktionsmedium kann vorteilhaft eine nach Abtrennung der Bisacrylate verbleibende Mutterlauge verwendet werden. Dadurch können die Wirtschaftlichkeit des Verfahrens und die erzielbaren Ausbeuten an Bisacrylat nochmals verbessert werden. Damit sich im Reaktionsmilieu im Laufe der Zeit keine Nebenprodukte ansammeln, können nach jedem Reaktionszyklus entsprechende Teile der Mutterlauge entnommen und separat aufgearbeitet werden.

Das Verfahren kann im allgemeinen so durchgeführt werden, daß man Bisphenol, Amin und Lösungsmittel vorlegt und das Acryloylhalogenid, gegebenenfalls verdünnt mit dem Lösungsmittel, unter Rühren zudosiert. Dabei fällt das Ammoniumhalogenid und in der Regel auch ein Teil des Bisphenol-bisacrylates aus. Da die Reaktion rasch verläuft, kann die Zudosierung bei effektiver Kühlung schnell erfolgen.

Die erfindungsgemäße Umsetzung kann auch kontinuierlich, beispielsweise in Umpumpreaktoren oder Rohrreaktoren mit Mischelementen, bei gleichzeitiger Dosierung der Bisphenollösung und des Acryloylhalogenids durchgeführt werden.

Anschließend kann die Reaktionslösung mit so viel Wasser versetzt werden, daß sich die Amin-Salze lösen und gleichzeitig auf 60°C, bevorzugt 50°C erwärmt werden, um die Bisacrylate gegebenenfalls vollständig in Lösung zu bringen. Man erhält dabei zwei klare, leicht trennbare Phasen. Sollte es erforderlich sein, kann die organische Phase durch einfache Filtration von Trübungen befreit werden. Das Bisacrylat wird nach Abkühlen der organischen Phase durch übliche Verfahren wie Filtrieren, Dekantieren oder Zentrifugieren abgetrennt.

Bei der hohen Reinheit der Rohprodukte kann eine Isolierung der Bisacrylate auch durch schonende rasche Verdampfung des Lösungsmittels, z.B. durch Sprühtrocknung, isoliert werden.

Die anfallende Mutterlauge kann für weitere Umsetzungen wieder eingesetzt werden. Die wäßrige Phase wird durch Zugabe von Alkali basisch gestellt, wobei das Amin als zweite Phase in gut abtrennbarer Form isoliert werden kann. Gegebenenfalls können letzte Reste des Amins mit dem Lösungsmittel extrahiert werden. Das Amin wird so praktisch quantitativ zurückgewonnen und kann nach azeotroper Entwässerung wieder eingesetzt werden.

Die Reaktionstemperatur liegt bei -10 bis +60°C, vorzugsweise -5 bis +50°C, besonders bevorzugt 0° bis 45°C.

Das Verhältnis der Reaktionspartner Bisphenol, Acrylsäurehalogenid und Amin ist etwa stöchiometrisch und beträgt 1:2:2 bis 1:2,2:2,4.

Zur Stabilisierung des Bisacrylates während der Umsetzung und Aufarbeitung können übliche Stabilisatoren wie Kupfersalze, Chinone, Phenole oder aromtische Amine zugesetzt werden. Genannt seien beispielsweise Kupfer-I-chlorid, Toluchinon, Hydrochinonmonomethylether und Phenothiazin. Man kann auch unter Luftzutritt arbeiten.

Die erfindungsgemäß erhaltenen Bisphenolbisacrylate sind rein und können für viele Zwecke ohne weitere Reinigung eingesetzt werden. Durch eine Umkristallisation aus geeigneten Lösungsmitteln, z.B. den bei der Umsetzung selbst verwendeten oder niederen Alkoholen wie Methanol, Ethanol oder Isopropanol können sie in hochreine Produkte übergeführt werden.

### Beispiele

### Beispiel 1

22,8 g (0,10 Mol) Bisphenol A werden unter Rühren in 21,0 g (0,21 Mol) Triethylamin gelöst und mit 100 bis 120 ml Cyclohexan verdünnt. Unter Eiskühlung und Rühren tropft man dazu 21,0 g (0,20 Mol) frisch destilliertes, mit 0,1 Gew.-% Hydrochinonmonomethylether versetztes Methacrylsäurechlorid in 30 min hinzu, wobei die Temperatur zwischen 15 bis 20°C gehalten wird. Nach einer Stunde wird eine Menge von 25 g Wasser zugegeben und die Mischung auf 40 bis 45°C erwärmt. Man trennt die wäßrige Phase ab und neutralisiert sie mit der hinreichenden Menge (mindestens 0,21 Mol) anorganischer Base wie Natrium-oder Kaliumhydroxid und trennt die aufschwimmende Aminphase ab. 20,5 g Amin werden so zurückgewonnen.

Nach gaschromatographischer Untersuchung enthält die organische Phase reines Bisphenol-A-bismethacrylat, das nur 0,2 Gew.-% des Bisphenol-A-monomethacrylates (Halbesters) enthält.

Durch Abkühlen der organischen Phase und Absaugen erhält man 27,8 g (d.s. 76 % d.Th.) Bisphenol-A-bisacrylat als farblose Kristalle (Fp.: 74-5°C).

### Beispiel 2

Man wiederholt Beispiel 1 und verwendet als Lösungsmittel die Cyclohexan-Mutterlauge aus Beispiel 1 unter Ergänzung etwaiger Verdunstungsverluste.

Nach der Reaktion und Abtrennung des Aminsalzes enthält die organische Phase Bisphenol-A-bismethacrylat und 0,3 Gew.-% des Halbesters. Nach Abkühlen, Absaugen und Trocknen erhält man 35,6 g (98 % d.Th.) an farblosen Kristallen (Fp.: 74-5°C).

### Beispiel 3

Man wiederholt Beispiel 1 mit 80 ml Cyclohexan und 38,0 g (0,20 Mol) Tributylamin statt (0,21 Mol) Triethylamin. Nach 5 h Reaktionszeit bei 20°C und Aufarbeitung wie in Beispiel 1 enthält die Cyclohexanlösung 1 Gew.-% Halbester. Man isoliert 27,6 g (76 % d.Th.) farblose Kristalle (Fp.: 74°C).

Eine Wiederholung dieses Beispiels analog Beispiel 2 mit der Mutterlauge erhöht die Ausbeute an Bisacrylat wie in Beispiel 2.

### Vergleichsbeispiel 1

22,8 g (0,10 Mol) Bisphenol A werden in einer Lösung aus 8,8 g (0,22 Mol) NaOH und 20 ml Wasser gelöst, mit 1,0 g Triethylamin als Katalysator, 80 ml Cyclohexan und dann in 2 h mit 22,0 g (0,21 Mol) Methacrylsäurechlorid bei 15 bis 20°C versetzt. Nach Aufarbeitung wie unter Beispiel 1 enthält die organische Phase außer Bisphenol A-bismethacrylat 0,5 Gew.-% Bisphenol A und 5 Gew.-% Monomethacrylat.

Man isoliert aus der Cyclohexanlösung analog Beispiel 1 27,8 g (d.s. 76 % d.Th.) an Bismethacrylat (Fp.: 48-56°C).

### Vergleichsbeispiel 2

Bei Wiederholung des Vergleichsbeispiels 1 findet man in der organischen Lösung neben dem Bismethacrylat 3 Gew.-% Bisphenol A und 4 % Monomethacrylat. Die Ergebnisse sind schwieriger reproduzierbar als nach dem erfindungsgemäßen Verfahren, weil die Zweiphasenreaktion in schwer zu übersehender Weise beeinflußt wird.

### Vergleichsbeispiel 3

Eine weitere Wiederholung des Vergleichsbeispiels 1 mit einem Überschuß von 20 Mol-% an Methacrylsäurechlorid und NaOH, um die Umwandlung in Bismethacrylat zu steigern, ergibt keine Verbesserung. Die Reaktionslösung enthält neben dem Bisacrylat 2 % Bisphenol A und 4 % Monoacrylat.

### Vergleichsbeispiel 4

Wird im Vergleichsbeispiel 1 statt Cyclohexan Methylenchlorid als Lösungsmittel verwendet, so kann das Reaktionsprodukt nur durch Eindampfen isoliert werden. Obwohl dies im Vakuum bei Raumtemperatur in Gegenwart von Stabilisatoren wie Toluchinon und Hydrochinonmonomethylether geschieht, erhält man auch bei wiederholten Versuchen immer einen teilweisen polymerisierten Eindampfrückstand.

### Vergleichsbeispiel 5

Wiederholt man Vergleichsbeispiel 1 und verwendet statt NaOH 32 g (0,30 Mol) pulverisiertes wasserfreies Natriumcarbonat und 2 g Triethylamin in Abwesenheit von Wasser und läßt 6 h bei 20 bis 30°C reagieren, so enthält die organische Phase neben Bisphenol A-Bismethacrylat 3 Gew.-% Bisphenol A und 4 Gew.-% Halbester. Das isolierte Produkt schmilzt bei 45 bis 53°C.

### Vergleichsbeispiel 6

114 g (0,50 Mol) Bisphenol A und 172 g (2,0 Mol) Methacrylsäure werden in 200 ml Benzol mit HCl-Gas gesättigt, das Gemisch langsam unter HCl-Einleiten zum Sieden erhitzt und 4 h gekocht. Nach Abkühlen isoliert man 70 g verunreinigtes Bisphenol A (Fp.: 130 bis 140°C). In der Mutterlauge kann gaschromatographisch kein Bisphenol-A-bismethacrylat gefunden werden.

## Patentansprüche

1. Verfahren zur Herstellung von Bisphenol-bisacrylaten durch Umsetzung von Bisphenolen mit Acrylsäurehalogeniden in Gegenwart von Basen, dadurch gekennzeichnet, daß man die Umsetzung in einem unpolaren Lösungsmittel in Anwesenheit eines tertiären Amins und in Abwesenheit einer wäßrigen Phase durchführt, wobei das Molverhältnis Bisphenol, Acrylsäurehalogenid und Amin 1:2:2 bis 1:2,2:2,4 beträgt, anschließend die Aminsalze durch Extraktion mit Wasser entfernt und die Bisphenolbisacrylate aus der organischen Phase kristallin gewinnt.

2. Verfahren nach Anspruch 1, dadurch gekennzeichnet, daß die tertiären Amine aliphatische Amine mit bis zu 12 C-Atomen sind.

3. Verfahren nach Anspruch 1, dadurch gekennzeichnet, daß Triethylamin eingesetzt wird.

4. Verfahren nach Anspruch 1, dadurch gekennzeichnet, daß Bisphenol A eingesetzt wird.

5. Verfahren nach Anspruch 1, dadurch gekennzeichnet, daß als Lösungsmittel Pentane, Hexane, Petrolether, Ligroin, Cyclohexan, Methylcyclohexan, Methylcyclopentan und Octane verwendet werden.

6. Verfahren nach Anspruch 1, dadurch gekennzeichnet, daß man die nach Abtrennung der Bisphenolbisacrylate erhaltene Mutterlauge erneut als Reaktionsmedium verwendet.

## Claims

1. Process for the preparation of bisphenol bisacrylates by reaction of bisphenols with acrylic acid halides in the presence of bases, characterised in that the reaction is carried out in a non-polar solvent in the presence of a tertiary amine and in the absence of an aqueous phase, the molar ratio of bisphenol, acrylic acid halide and amine being 1:2:2 to 1:2.2:2.4, the amine salts are then removed by extraction with water and the bisphenol bisacrylates are isolated as crystals from the organic phase.

2. Process according to Claim 1, characterised in that the tertiary amines are aliphatic amines having up to 12 C atoms.

3. Process according to Claim 1, characterised in that triethylamine is employed.

4. Process according to Claim 1, characterised in that bisphenol A is employed.

5. Process according to Claim 1, characterised in that pentanes, hexanes, petroleum ether, ligroin, cyclohexane, methylcyclohexane, methylcyclopentane and octanes are used as the solvent.

6. Process according to Claim 1, characterised in that the mother liquor obtained after the bisphenol bisacrylates have been separated off is used again as the reaction medium.

## Revendications

1. Procédé de préparation de bis-acrylates de bis-phénols par réaction de bis-phénols avec des halogénures d'acides acryliques en présence de bases, caractérisé en ce que l'on effectue la réaction dans un solvant non polaire en présence d'une amine tertiaire et en l'absence d'une phase aqueuse, avec des proportions molaires de 1:2:2 à 1:2,2:2,4 entre le bis-phénol, l'halogénure d'acide acrylique et l'amine, on élimine ensuite les sels d'amines par extraction à l'eau et on isole les bis-acrylates de bis-phénols de la phase organique à l'état cristallisé.

2. Procédé selon la revendication 1, caractérisé en ce que les amines tertiaires sont des amines aliphatiques contenant jusqu'à 12 atomes de carbone.

3. Procédé selon la revendication 1: caractérisé en ce que l'on utilise la triéthylamine.

4. Procédé selon la revendication 1, caractérisé en ce que l'on utilise le bis-phénol A.

5. Procédé selon la revendication 1, caractérisé en ce que l'on utilise en tant que solvants des pentanes, des hexanes, des éthers de pétrole, des essences minérales légères, du cyclohexane, du méthylcyclohexane, du méthylcyclopentane ou des octanes.

6. Procédé selon la revendication 1, caractérisé en ce que après séparation des bis-acrylates de bis-phénols, on réutilise les liqueurs mères obtenues en tant que milieu de réaction.
